(19) Europäisches Patentamt
European Patent Office
Office européen des brevets

(11) Publication number: 0 144 996
B1

(12) # EUROPEAN PATENT SPECIFICATION

(45) Date of publication of patent specification: 28.02.90

(21) Application number: 84114974.3

(22) Date of filing: 08.12.84

(51) Int. Cl.⁵: **C 07 D 471/10**, C 07 D 491/107, C 07 D 495/10, C 07 D 221/20, C 07 D 471/20, C 07 D 491/20, C 07 D 495/20, A 61 K 31/435, A 61 K 31/495, A 61 K 31/50, A 61 K 31/505 // (C07D471/10, 221:00, 221:00),(C07D491/20, 311:00, 221:00, 221:00),(C07D491/20, 307:00, 221:00, 221:00),(C07D491/20, 303:00, 221:00, 221:00),(C07D471/20, 221:00, 221:00), C07D221:00

(54) **Substituted spiro pyridine derivatives, pharmaceutical compositions containing them and processes for the preparation of such compounds and compositions.**

(30) Priority: 14.12.83 US 561416
15.08.84 US 641076

(43) Date of publication of application:
19.06.85 Bulletin 85/25

(45) Publication of the grant of the patent:
28.02.90 Bulletin 90/09

(84) Designated Contracting States:
AT BE CH DE FR IT LI LU NL SE

(56) References cited:
EP-A-0 018 139
EP-A-0 092 786
FR-A-2 255 068
GB-A- 855 022

(73) Proprietor: SCHERING CORPORATION
2000 Galloping Hill Road
Kenilworth New Jersey 07033 (US)

(72) Inventor: Blythin, David John
487 Mountain Avenue
North Caldwell New Jersey 07006 (US)

(74) Representative: Ritter, Stephen David et al
Mathys & Squire 10 Fleet Street
London EC4Y 1AY (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 144 996 B1

## Description

The invention relates to compounds of the general formula I

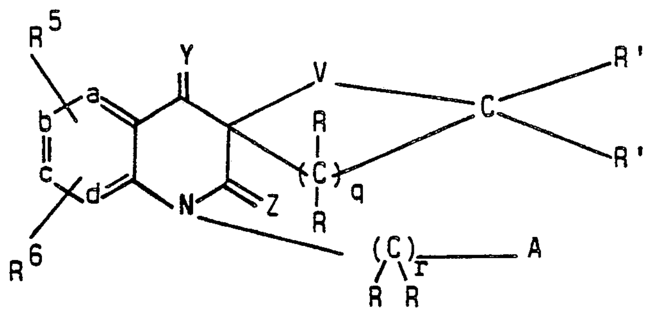

I

wherein

two of the ring atoms a, b, c and d may be CH or N and the remaining two atoms represent CH;

Y and Z independently represent O or S;

V represents O, $S(O)_n$, $N—R^8$ or

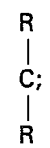

each R independently represents hydrogen, $C_1$ to $C_6$ alkyl, $CH_2OH$, $COR_7$ (wherein $R^7$ represents hydrogen or $C_1$ to $C_6$ alkyl) or hydroxy, with the proviso that only one hydroxy group can be attached to one carbon atom;

each R′ independently is as defined for R above, except that when V represents O, $S(O)_n$ or $N—R^8$, R′ may not be hydroxy;

$R^5$ and $R^6$ may be the same or different and are hydrogen, alkyl having from 1 to 6 carbon atoms, halogen, nitro, alkoxy having from 1 to 6 carbon atoms, trifluoromethyl, alkylthio having 1 to 6 carbon atoms or cyano; $R^8$ is hydrogen, alkyl having from 1 to 6 carbon atoms, carboxylic acyl having from 2 to 7 carbon atoms, alkylsulfonyl having from 1 to 6 carbon atoms, carboalkoxy having from 2 to 7 carbon atoms, $CONH_2$, phenyl or pyridyl of which the last two may be substituted with up to three substituents, Q, whereby each Q independently is hydroxy, alkyl having from 1 to 6 carbon atoms, halogen, nitro, alkoxy having from 1 to 6 carbon atoms, trifluoromethyl, cyano, cycloalkyl, having from 3 to 7 carbon atoms, alkenyloxy having from 3 to 6 carbon atoms, alkynyloxy having from 3 to 6 carbon atoms, $S(O)_n—R^a$ [wherein n is defined herein and $R^a$ is alkyl having from 1 to 6 carbon atoms], $NHSO_2R^a$ [wherein $R^a$ is defined herein], $NHSO_2CF_3$, $SO_2NH_2$, $COR^b$ [wherein $R^b$ is OH, $NH_2$ or $OR^a$ (wherein $R^a$ is defined herein)], $O—B—COR^a$ [wherein B is alkylene having from 1 to 4 carbon atoms and $R^b$ is defined herein], or $NHCOR^c$ [wherein $R^c$ is hydrogen, alkyl having from 1 to 6 carbon atoms, alkoxy having from 1 to 6 carbon atoms, $COR^d$ (wherein $R^d$ is hydroxy or alkoxy having from 1 to 6 carbon atoms) or $NHR^e$ (wherein $R^e$ is hydrogen or alkyl having 1 to 6 carbon atoms)];

n is 0, 1 or 2;

r is 0, 1 or 2;

q is an integer of from 1 to 5; and

A is phenyl, naphthyl, indenyl, indanyl, pyridyl, pyrimidyl, pyrazinyl, furyl, thienyl, imidazolyl, thiazolyl or oxazolyl, any of which may be substituted with up to three substituents Q as defined herein above, and salts and solvates of such compounds.

A preferred subgenus of compounds is that wherein Y and Z are both oxygen.

Preferably a, b and c represent CH and d is either CH or N. Alternatively, a and d are both N and b and c are both CH. A further preferred feature is that r is zero, i.e. the group A is directly attached to the ring-nitrogen atom. q is preferably 2, 3 or 4, V most preferably is $CH_2$ or O and $R^8$ and $R^6$ both hydrogen.

Particularly preferred are compounds of the structural formula III:

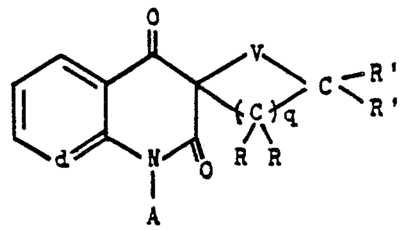

III

wherein d is CH or N, q is 2, 3 or 4 and A, R and R′ are as defined above.

Similar spiro[cyclopentane]-quinolinediones are described in Chem. Pharm. Bull., 17, 1290 (1969) and in Bull. Soc. Chem. Fr., 364 (1968). The references do not describe pharmaceutical uses for these compounds. EP—A—0 092 786 and EP—A—018 139 describe naphthiridine and pyridopyrimidine compounds which are structurally distinct from the spiro pyridine derivatives herein.

When utilized herein and in the appended claims the below listed terms, unless specified otherwise, are defined as follows:

halogen — fluorine, chlorine, bromine and iodine;

alkyl and alkoxy — comprised of straight and branched carbon chains containing from 1 to 6 carbon atoms;

alkenyloxy — comprised of straight and branched carbon atom chains containing from 3 to 6 carbon atoms and comprising a carbon to carbon double bond; and

alkynyloxy — comprised of straight and branched carbon chains containing from 3 to 6 carbon atoms and comprising a carbon to carbon triple bond.

The compounds of the invention include a

$-(C)_r$ and a $-(C)_q-$
/ \ / \
R R R R

substituent wherein the R and R′ groups may vary independently. Thus, for example, when r equals 2 the following patterns of substitution (wherein hydrogen and $CH_3$ are used to represent any substituents R) are contemplated: $-C(CH_3)_2CH_2-$, $-CH_2C(CH_3)_2-$, $-CH_2CH(CH_3)-$, $-CH(CH_3)CH_2-$, $-(C(CH_3)H)_2-$ and the like. In addition when r equals 2, substituents such as $-C(CH_3)_2CH(C_2H_5)-$, $-CH(CH_3)CH(C_2H_5)-$, $-CH(i-C_3H_7)CH(C_2H_5)-$ are also contemplated.

It would be obvious to one of ordinary skill in the art that due to problems of stability there are limitations involving the R and $R^1$ groups. One limitation is that neither R can be a hydroxy group attached to the carbon alpha to the ring nitrogen. Another limitation is that the R and $R^1$ groups cannot both be hydroxy groups attached to the same carbon atoms. Preferably R and $R^1$ independently are hydrogen methyl, ethyl, propyl, isopropyl, n-butyl or iso-butyl.

Certain compounds of the invention may exist in isomeric forms. The invention contemplates all such isomers both in pure form and in admixture, including racemic mixtures. In structural formulas I, II and III herein, when V represents the hetero atom in the spiro ring, V is attached directly to the spiro carbon atom, i.e. the carbon atom identified as number 3 in structural formula I.

The compounds of the invention of formula I can exist in unsolvated as well as solvated forms, including hydrated forms. In general, the solvated forms, with pharmaceutically acceptable solvents such as water, ethanol and the like are equivalent to the unsolvated forms for purposes of the invention.

The compounds of formula I may be prepared by a process which is characterized in that a compound of the general formula IV

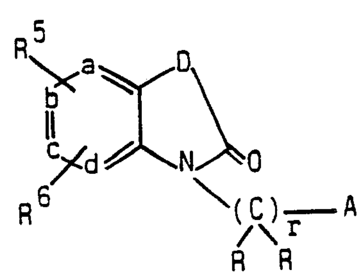

IV

wherein a, b, c, d, R, $R^5$, $R^6$, A and r are as defined above and D represents either of the groups

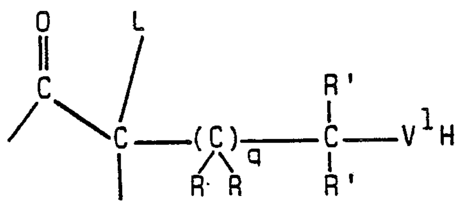 or 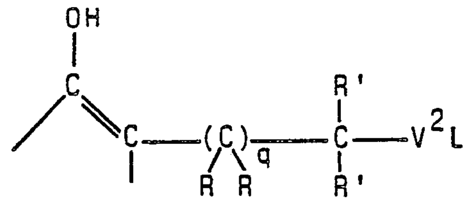

VI VII

wherein L is a good leaving group, $V^1$ is O, $S(O)_n$ or

$N—R^8$, $V^2$ is 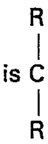

and R, R′ and q are as defined above, is subjected to an intramolecular condensation yielding a compound of formula I wherein both Y and Z are oxygen, followed, if desired, by replacing one or both of the oxygen atoms represented by Y and Z with sulfur.

Treatment of compound IV with an organic base such as triethylamine or 1,8-diazabicyclo [5.4.0] undec-7-ene, DBU [Angew. Chem., Internat. Ed., *6* 76 (1967)] in a nonreactive solvent such as chloroform will produce the compounds of the invention having structural formula I, wherein Y and Z are both oxygen.

For purposes of the invention a "leaving group" is defined as a substituent which may be displaced and carry a negative charge. Examples of such substituents are bromide, iodide, trifluoroacetoxy, p-toluenesulfonyloxy, methanesulfonyloxy and the like. The preferred leaving group is bromine.

The starting compounds of formula IV may be prepared by methods known in the art. Thus, for example compounds having structural formula IV wherein D represents the group VII and L is Br may be prepared by known methods from the corresponding hydroxy compound by treatment with concentrated HBr (e.g. 48% HBr). Other desired leaving group substituents, L, may be prepared by similar methods.

Exemplary of such starting materials for preparing compounds having structural formula IV are 2-anilino nicotinic acids which may be prepared, for example, as described in United States Reissue Patent 26,655; and 2-phenylamino-3-pyrazine carboxylate esters which may be prepared substantially as exemplified herein starting with a 2-amino-3-pyrazine carboxylate ester. 2-Anilino-3-pyrazine carboxylic acid is a known compound, C.A., *75,* 20154e (1971), which may be esterified by standard procedures. Compounds of formula IV may be obtained from such compounds by following the processes described in European Patent Application No. 0092786.

Compounds having structural formula IV wherein D represents the group VI, L is bromine and $V^1$ is oxygen may be prepared by treatment of the corresponding non-brominated compounds with a solution of bromine in chloroform. Other compounds having structural formula IV wherein leaving group substituent L is bromine or iodine and $V^1$ is a heteroatom or group may be prepared by methods known to those of skill in the art.

The compounds having structural formula IV without the leaving group substituent L may be prepared by known methods from known starting materials. Such methods are described, for example, in the preparative examples.

The compounds having structural formula I wherein Y and/or Z are oxygen may be converted to the corresponding compounds wherein Y and/or Z are sulfur by known methods. For example treatment with Lawesson's Reagent [2,4-bis(4-methoxyphenyl)-1,3-dithia-2,4-diphosphetane-2,4-disulfide] in hot toluene will effect this conversion.

The compounds of this invention can be used to treat allergy-caused diseases and their preferred use is for treating allergic chronic obstructive lung diseases. Chronic obstructive lung disease as used herein means disease conditions in which the passage of air through the lungs is obstructed or diminished such as is the case in asthma, bronchitis and the like.

The anti-allergy method of this invention is identified by tests which measure a compound's inhibition of anaphylactic bronchospasm in sensitized guinea pigs having antigen-induced broncho-constriction. For example, the compound 1-phenyl-3′,4′,5′,6′-tetrahydro-spiro[1,8]-naphthyridine 3,2′-(2H)pyran-2,4-dione was found to inhibit anaphylactic bronchospasms in such test procedure when given at an oral dose of 5 mg/kg. Said compound was also found to inhibit allergen-induced histamine release from guinea pig and human sensitized tissue. The compounds are effective non-adrenergic, non-anticholinergic anti-anaphylactic agents. When administered orally they are active at doses from about 0.1 to 50 mg/kg of body weight; when administered parenterally, e.g., intravenously, the compounds are active at dosages of from about 0.01 to 5 mg/kg body weight; when administered by inhalation (aerosol or nebulizer) the compounds are active at dosages of about 0.1 to 5 mg per puff, and one to four puffs may be taken every 4 hours.

The compounds of this invention are also useful for the treatment of inflammation; thus, they are

useful for the treatment of: arthritis, bursitis, tendonitis, gout and other inflammatory conditions. The anti-inflammatory use of the compounds of the present invention may be demonstrated by the Reversed Passive Arthus Response technique as set forth below using male Lewis inbred albino rats (Charles River) weighing 180—200 grams. The potency of the compounds is determined using indomethacin as the standard. On the basis of the test results, a dosage range of 5 mpk to about 50 mpk in divided doses taken at about 4 hour intervals is recommended.

The dosage to be administered and the route of administration depends upon the particular compound used, the age and general health of the patient and the severity of the inflammatory conditions. Thus, the dose ultimately decided upon must be left to the judgement of a trained health-care practitioner.

Reversed Passive Arthus Response (APAR)

Animals, Materials and Methods

Male Lewis inbred albino rats weighing 180—200 grams obtained from Charles River Breeding Laboratories are used in these experiments. The rats are housed 3 animals/cage and food and water are allowed *ad libitum.* The animals are numbered 1—3 in each cage and color marked for identification purposes.

Drug and Reagent Preparation

All reagents and drugs are prepared just prior to the study. Crystallized and lyophilized bovine serum albumin (BSA), obtained from Sigma Chemical Company, is solubilized without shaking in cold sterile pyrogen-free saline (10 mg/ml). Lyophilized anti-bovine serum albumin (IGG fraction), obtained from Cappel Laboratories, is suspended in sterile distilled water and diluted with cold pyrogen free saline (PFS) just prior to use. The final concentration of anti-bovine serum albumin is 0.5 mg/ml of PFS. Both BSA and anti-BSA solutions are iced during use. Drugs are suspended or solubilized in an aqueous solution of methyl cellulose (MC) with a homogenizer just prior to administration.

Drug Administration and Induction of Inflammation

Groups of animals (6/group) are dosed with drug in MC by gavage once daily for 3 days. The last dose is administered one hour prior to sensitization with BSA. Controls are given MC alone and a drug-standard is usually included in each assay for verification purposes. Drugs are prepared so as to provide a dose for a 200 gram animal which is equivalent to the mg/kg dose for the experiment. Thus each rat receives an oral dose in a volume of approximately 2.0 cc. One hour after the last dose the animals are lightly anesthetized with ether and "sensitized" by injection into the penile vein with 0.2 ml of PFS containing 1.0 mg of BSA. One hour later, the animals are "challenged" in the right rear paw with subplantar injections of 0.2 ml of PFS containing 0.1 mg of anti-BSA. Immediately after the subplantar injection, the right paw is dipped (up to the lateral maleolus) into the mercury well of a plethysmograph. The volume of mercury displaced is converted to weight and recorded. This value is considered to be the control reading for the animal. Paw volumes are also recorded with a plethysmograph during the development of the inflammation at 2 and 4 hours post-challenge.

Results

Results are expressed by the change in paw volume (Δ paw volume) from the control reading for each animal to that recorded 2 and 4 hours post-challenge. All drug-treated groups are compared to the MC control for significant differences with an analysis of variance. Differences from control in drug-treated groups are expressed as percent change from control.

The compounds of this invention are also useful in the treatment of peptic ulcers. They display chemotherapeutic activity which enables them to relieve the symptoms of peptic ulcer disease and stress ulceration, and promote healing of gastric and/or duodenal ulcers. The antiulcer activity of the compounds of this invention is identified by tests which measure the cytoprotective effect in rats. The compounds are also useful as conjunctive therapeutic agents for coadministration with such anti-inflammatory/analgesic agents as aspirin, indomethacin, phenylbutazone, ibuprofen, naproxen, tolmetin and other agents. The compounds of this invention prevent the untoward side effects of irritation and damage to the gastrointestinal tract caused by such agents.

The compounds of this invention are evaluated for their antiulcer activity characteristics by standard biological testing procedures.

In cytoprotective tests in rats in which ethanol is employed to induce gastrointestinal damage, the compounds of this invention are found to be effective at doses of about 0.5—50 mg/kg of body weight per day. Preferably the total dosages are administered in divided doses per day.

When administered parenterally, e.g. intraveneously, the compounds are administered at a dosage range of about 0.05—5 mg/kg of body weight in single or multiple daily doses.

To treat peptic ulcer diseases, and prevent and treat drug-induced gastric ulceration, the active compounds of this invention can be administered in unit dosage forms such as tablets, capsules, pill, powders, granules, sterile parenteral solutions or suspensions, suppositories, mechanical delivery devices, e.g. transdermal, and the like.

For preparing pharmaceutical compositions from the compounds described by this invention, inert,

pharmaceutically acceptable carriers can be either solid or liquid. Solid form preparations include powders, tablets, dispersible granules, capsules, cachets and suppositories. A solid carrier can be one or more substances which may also act as diluents, flavoring agents, solubilizers, lubricants, suspending agents, binders or tablet disintegrating agents; it can also be an encapsulating material. In powders, the carrier is a finely divided solid which is in admixture with the finely divided active compound. In the tablet the active compound is mixed with carrier having the necessary binding properties in suitable proportions and compacted in the shape and size desired. The powders and tablets preferably contain from 5 or 10 to about 70 percent of the active ingedient. Suitable solid carriers are magnesium carbonate, magnesium stearate, talc, sugar, lactose, pectin, dextrin, starch, gelatin, tragacanth, methylcellulose, sodium carboxymethylcellulose, a low melting wax, cocoa butter and the like. The term "preparation" is intended to include the formulation of the active compound with encapsulating material as carrier providing a capsule in which the active component (with or without other carriers) is surrounded by carrier, which is thus in association with it. Similarly, cachets are included. Tablets, powders, cachets and capsules can be used as solid dosage forms suitable for oral administration.

For preparing suppositories, a low melting wax such as a mixture of fatty acid glycerides or cocoa butter is first melted, and the active ingredient is dispersed homogeneously therein as by stirring. The molten homogeneous mixture is then poured into convenient sized molds, allowed to cool and thereby solidify.

Liquid form preparations include solutions, suspensions and emulsions. As an example may be mentioned water or water-propylene glycol solutions for parenteral injection. Liquid preparations can also be formulated in solution in aqueous polyethylene glycol solution. Aqueous solutions suitable for oral use can be prepared by adding the active component in water and adding suitable colorants, flavors, stabilizing, sweetening, solubilizing and thickening agents as desired. Aqueous suspensions suitable for oral use can be made by dispersing the finely divided active component in water with viscous material, i.e., natural or synthetic gums, resins, methylcellulose, sodium carboxymethylcellulose and other well-known suspending agents.

Also included are solid form preparations which are intended to be converted, shortly before use, to liquid form preparations for either oral or parenteral administration. Such liquid forms include solutions, suspensions and emulsions. These particular solid form preparations are most conveniently provided in unit dose form and as such are used to provide a single liquid dosage unit. Alternately, sufficient solid may be provided so that after conversion to liquid form, multiple individual liquid doses may be obtained by measuring predetermined volumes of the liquid form preparation as with syringe, teaspoon or other volumetric container. When multiple liquid doses are so prepared, it is preferred to maintain the unused portion of said liquid doses at low temperature (i.e., under refrigeration) in order to retard possible decomposition. The solid form preparations intended to be converted to liquid form may contain, in addition to the active material, flavorants, colorants, stabilizers, buffers, artificial and natural sweeteners, dispersants, thickeners, solubilizing agents and the like. The solvent utilized for preparing the liquid form preparation may be water, isotonic water, ethanol, glycerine, propylene glycol and the like as well as mixtures thereof. Naturally, the solvent utilized will be chosen with regard to the route of administration, for example, liquid preparations containing large amounts of ethanol are not suitable for parenteral use.

Preferably, the pharmaceutical preparation is in unit dosage form. In such form, the preparation is subdivided into unit doses containing appropriate quantities of the active component. The unit dosage form can be a packaged preparation, the package containing discrete quantities of preparation, for example, packeted tablets, capsules and powders in vials or ampoules. The unit dosage form can also be a capsule, cachet or tablet itself or it can be the appropriate number of any of these in packaged form.

The quantity of active compound in a unit dose of preparation may be varied or adjusted from 1 mg to 100 mg according to the particular application and the potency of the active ingredient. The compositions can, if desired, also contain other therapeutic agents.

The dosages may be varied depending upon the requirements of the patient, the severity of the condition being treated and the particular compound being employed. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day if desired.

Preparative Example 1

4-Hydroxy-3-(4-hydroxybutyl)-1-phenyl-1,8-naphthyridin-2(1H)-one

A mixture of methyl 2-phenylamino-nicotinate (100 g), ε-caprolactone (1000 g) and potassium t-butoxide (200 g) was stirred at room temperature, in a nitrogen atmosphere, for ½ hr. It was heated at 45°C for 1 hr then at 85°C for 2 hrs and finally at 105°C for 3 hrs.

The hot mixture was poured carefully into 8L of 5% KOH solution and was stirred overnight.

The mixture was extracted with 2L of ether and the aqueous phase was retained. It was extracted again with a fresh 2L of ether. The clear aqueous phase was adjusted to pH 4.5 with conc. HCl to yield a white solid which was filtered off, washed with water and dried to yield 4-hydroxy-3-(4-hydroxybutyl)-1-phenyl-1,8-naphthyridin-2(1*H*)-one, m.p. 205.5—206.5°C (from isopropanol).

By substituting the relevant ester and lactone in this preparative example intermediates to many other compounds of the invention may be prepared.

Preparative Example 2

4-Hydroxy-3-(3-hydroxypropyl)-1-phenyl-1,8-naphthyridin-2(1H)one

Methyl 2-phenylamino-nicotinate (25 g) was dissolved in w-valerolactone (240 g) with stirring in an atmosphere of nitrogen. To the resulting solution was added potassium t-butoxide (50 g) and the mixture was stirred at room temperature for ½ hr. It was then heated to 100°C for 3 hrs after which time it was poured into 1L of 5% NaOH solution and stirred overnight.

The mixture was extracted (2×) with 1L of ether then the aqueous layer was adjusted to pH 4.5 with conc. HCl. The solid which separated was filtered off, washed with water and dried to yield 4-hydroxy-3-(3-hydroxypropyl)-1-phenyl-1,8-naphthyridin-2(1*H*)-one, m.p. 218—220°C.

By utilizing the correspondingly substituted starting materials in the procedures of preparative examples 1 or 2, the following compounds were obtained:

1-(4-chlorophenyl)-4-hydroxy-3-(3-hydroxypropyl)-[1,8]-naphthyridin-2(1*H*)-one, m.p. 249.5—251°C;
4-hydroxy-3-(3-hydroxypropyl)-1-(4-methylphenyl)-[1,8]-naphthyridin-2(1*H*)-one, m.p. 227—228°C;
4-hydroxy-3-(3-hydroxypropyl)-1-(4-methylphenyl)-[1,8]-naphthyridin-2(1*H*)-one, m.p. 229—231°C;
1-(3,4-dichlorophenyl)-4-hydroxy-3-(3-hydroxypropyl)-[1,8]-naphthyridin-2(1*H*)-one, m.p. 230—232°C;
1-(4-chlorophenyl)-4-hydroxy-3-(4-hydroxybutyl)-[1,8]-naphthyridin-2(1*H*)-one, m.p. 238—240°C;
4-3-(4-hydroxybutyl)-1-(4-methylphenyl)-[1,8]-naphthyridin-2(1*H*)-one, m.p. 186—188°C;
4-hydroxy-3-(3-hydroxybutyl)-1-(4-methoxyphenyl)-[1,8]-naphthyridin-2(1*H*)-one, m.p. 237—239°C;
1-(3,4-dichlorophenyl)-4-hydroxy-3-(4-hydroxybutyl)-[1,8]-naphthyridin-2(1*H*)-one, m.p. 188—190°C;
1-(3-chlorophenyl)-4-hydroxy-3-(4-hydroxybutyl)-[1,8]-naphthyridin-2(1*H*)-one, m.p. 176—178°C;
4-hydroxy-3-(4-hydroxybutyl)-1-(3-methoxyphenyl)-[1,8]-naphthyridin-2(1*H*)-one, m.p. 217—219°C;
4-hydroxy-3-(4-hydroxybutyl)-1-phenyl-quinolin-2(1*H*)-one, m.p. 165.5—158°C.

Preparation Example 3

Ethyl 5-(4-hydroxy-2-oxo-1-phenyl-1H-[1,8]naphthyridin-3-yl)pentanoate

Methyl 2-phenylaminonicotinate (8.5 g) was dissolved with stirring in diethyl pimelate (80 ml) in an atmosphere of nitrogen. To the mixture was added potassium t-butoxide (13 g) and the mixture was stirred at room temperature for 1 hr. It was then heated to 135—140°C for 6 hours after which time it was poured into water. The aqueous layer was extracted with methylene chloride and then adjusted to pH 4.5 with conc. HCl. Solid sodium chloride was added after which the solid was filtered off, washed with water and dried, m.p. 168—169°C.

By substituting diethyl suberate for diethylpimelate in the above procedure, ethyl 6-(4-hydroxy-2-oxo-1-phenyl-1H-[1,8]naphthyridin-3-yl)-hexanoate, m.p. 167—168°C was obtained.

Preparative Example 4

4-Hydroxy-3-(5-hydroxypentyl)-1-phenyl-1,8-naphthyridin-2(1H)-one

To a suspension of ethyl 5-(4-hydroxy-2-oxo-1-phenyl-1*H*-[1,8]-naphthyridin-3-yl]pentanoate (1 g) (prepared as in preparative example 3) in dry dioxane (50 ml) in an atmosphere of nitrogen is added lithium borohydride (0.34 g). The mixture is stirred at room temperature for 20 min then it is heated to 60°C for 16 hrs.

The product is poured into water, adjusted to pH 4.5 with acetic acid and the resulting solid is filtered off. The solid is washed with water and dried to yield 4-hydroxy-3-(5-hydroxypentyl)-1-phenyl-1,8-naphthyridin-2-(1H)-one.

Preparative Example 5

3-(4-Bromobutyl)-4-hydroxy-1-phenyl-1,8-naphthyridin-2(1H)-one

In 47% HBr (100 ml) was dissolved 4-hydroxy-3-(4-hydroxybutyl)-1-phenyl-1,8-naphthyridin-2(1*H*)-one (5 g) in an atmosphere of nitrogen at room temperature. After 1 hr the solution was heated to 90°C and it was kept there for 6 hrs.

After cooling, the product was poured into 1L of $H_2O$ and the pH was adjusted to 5 with potassium acetate. After stirring for 5 minutes, the solid was filtered off, washed with water and dried to yield 3-(4-bromobutyl)-4-hydroxy-1-phenyl-1,8-naphthyridin-2(1*H*)-one, m.p. 194—196°C.

By substituting the appropriate starting materials and using the procedure as above, the following additional compounds were obtained:

3-(4-bromobutyl)-4-hydroxy-1-(3,4-dichlorophenyl)-1,8-naphthyridin-2(1*H*)-one, m.p. 169—171°C;
3-(4-bromobutyl)-4-hydroxy-1-(4-chlorophenyl)-1,8-naphthyridin-2(1*H*)-one, m.p. 228—230°C;
3-(4-bromobutyl)-1-(3-hydroxyphenyl)-4-hydroxy-1,8-naphthyridin-2(1*H*)-one, m.p. 214—216°C;
3-(4-bromobutyl)-1-(3-methoxyphenyl)-4-hydroxy-1,8-naphthyridin-2(1*H*)-one, m.p. 179.5—181°C;
3-(4-bromobutyl)-1-(3-chlorophenyl)-4-hydroxy-1,8-naphthyridin-2(1*H*)-one, m.p. 195.5—197°C;
3-(4-bromobutyl)-4-hydroxy-1-phenyl-quinolin-2(1*H*)-one, m.p. 206.5—208°C.

Preparative Example 6

Methyl-2-phenylamino-3-pyrazine carboxylate

(A) Methyl 2-bromo-3-pyrazine carboxylate:

To a stirred mixture of 12.7 g of methyl 2-amino pyrazine carboxylate and 47 ml of 48% hydrobromic acid there was added, dropwise, 12.6 ml of bromine keeping the temperature at 0°. A solution of sodium nitrite in 60 ml of water was then added dropwise at 0°C and the reaction mixture stirred for 15 mins. The reaction mixture was basified to pH 8 with 14.4 g of sodium bicarbonate and extracted with ethyl acetate and again with chloroform. The organic layers were dried over magnesium sulfate, filtered and concentrated to a yellow oil. Recrystallization from ether-hexane yielded the product, m.p. 43—45°C.

(B) Methyl 2-phenylamino-3-pyrazine carboxylate:

A mixture of 9.5 g of methyl 2-bromo-3-pyrazine carboxylate, 8.2 g of aniline, 0.5 g of p-toluene sulfonic acid and 100 ml of water was stirred and refluxed for two hours. The reaction mixture was poured on ice, extracted with ethyl acetate, the organic extracts were dried and concentrated to yield an oil. The crude residue was eluted on a silica gel column with ethyl acetate-hexane (1:2) yielding the product of this example as yellow solid, m.p. 72—75°C.

Preparative Example 7

3-(2-Hydroxyethyl)-4-hydroxy-1-phenyl-1,8-naphthyridin-2(1H)-one

To a solution of 6.8 g of methyl 2-phenylamino-3-pyridine carboxylate in 60 ml of gamma-butyrolactone there was added, under nitrogen, 13.4 g of potassium tertiary butoxide. The reaction mixture was heated and stirred for one hour at 95°C, poured on ice and stirred overnight. The mixture was extracted with ether, the aqueous layer acidified with acetic acid to pH 4.5 and the product was collected by filtration. Recrystallization from chloroform, acetone, isopropanol yielded the product of this example as a colorless solid, m.p. 235—236°C.

Preparative Example 8

3,9-Dihydro-9-phenyl-furo[2,3-b][1,8]-naphthyridin-4(2H)-one

A solution of 4-hydroxy-3-(2-hydroxyethyl)-1-phenyl-1,8-naphthyridin-2(1*H*)-one in Eaton's Reagent (10% $P_2O_5$ in methane sulfonic acid; 40 ml) was stirred in an atmosphere of nitrogen and was heated to 70°C for 2 hr. After cooling, the product was poured into water, adjusted to pH 4 with $NaHCO_3$, filtered, washed with water, air dried and recrystallized from isopropanol with decolorization to yield the product, m.p. 245—247°C.

Example 1

1'-Phenylspiro[cyclopentane-1,3'-(1,8)-naphthyridine]-2',4'-(1'H)-dione

A suspension of 3-(4-bromobutyl)-4-hydroxy-1-phenyl-1,8-naphthyridin-2(1*H*)-one (5 g) in methylene chloride (350 ml), in an atmosphere of nitrogen, was stirred at room temperature and to it was added triethylamine (4.1 ml). The mixture was stirred at room temperature for 16 hrs. Water (300 ml) was added and the aqueous layer was back-extracted with methylene chloride. The combined organic layers were washed with water, dried ($Na_2SO_4$), filtered and evaporated to yield a solid which was recrystallized from isopropanol to yield 1'-phenylspiro[cyclopentane-1,3'-(1,8)naphthyridine]-2',4'-(1*H*)dione, m.p. 178—179°C.

1'-(4-methylphenyl)spiro[cyclopentane-1,3'-(1,8)naphthyridine]-2',4'-(1'*H*)-dione; m.p. 177—179.5°C;

1'-(4-chlorophenyl)spiro[cyclopentane-1,3'-(1,8)naphthyridine]-2',4'-(1'*H*)-dione; m.p. 181.5—183°C;

1'-(3,4-dichlorophenyl)spiro[cyclopentane-1,3'-(1,8)naphthyridine]-2',4'-(1'*H*)-dione; m.p. 143—145.5°C;

1'-(3-chlorophenyl)spiro[cyclopentane-1,3'-(1,8)naphthyridine]-2',4'-(1'*H*)-dione; m.p. 165—165°C;

1'-(3-methoxyphenyl)spiro[cyclopentane-1,3'-(1,8)naphthyridine]-2',4'-(1'*H*)-dione; m.p. 159—160.5°C;

1'-(3-hydroxyphenyl)spiro[cyclopentane-1,3'-(1,8)naphthyridine]-2',4'-(1'*H*)-dione; m.p. 218—220°C;

1'-phenylspiro[cyclopentane-1,3'-quinoline]-2',4'-(1'*H*)-dione; m.p. 166—168°C.

In general, by following the procedures described in Preparative Examples 1, 4, 5 or 6, or an art-recognized modification thereof, using lactones with desired substituents, other intermediates may be prepared which are useful for preparing products of the invention by the method of Example 1.

Example 2

1'-Phenylspiro[cyclohex-3-ene]-1,3'-[1,8]naphthyridine]-2',4'-(1'H)-dione

A. 4-Hydroxy-3-(5-hydroxy-3-pentynyl)-1-phenyl-1,8-naphthyridin-2(1H)-one

A solution of 3,9-dihydro-9-phenyl-furo[2,3-b]-[1,8]naphthyridin-4[2*H*]-one (prepared as in Preparative Example 8) in dimethylsulfoxide/tetrahydrofuran (DMSO/THF) is stirred and cooled to *ca.* 0°C while to it is added a pre-formed solution of the sodium salt of the tetra-hydropyranyl (THP) ether of propargyl alcohol ($Na^+$ $^-$Cj—$CH_2$—O—THP) which is prepared from the THP ether of proparagyl alcohol and an equivalent amount of dimsyl sodium in DMSO/THF. Dimsyl sodium is prepared by refluxing the desired amount of sodium hydride in DMSO/THF (1:10) until reaction is complete.

The reaction is allowed to warm up until reaction is observed, (monitor by t.l.c.). When complete the reaction is made sufficiently acidic to remove the protecting group, and the product is isolated.

B. (Z)-4-Hydroxy-3-(5-hydroxy-3-pentenyl)-1-phenyl-1,8-naphthyridin-2(1H) one.

The acetylene (from part A) is dissolved in methanol containing 2% by weight (of the acetylene) of 5% palladium on barium sulfate which also contains pure quinoline in amount equal to the weight of the catalyst. The mixture is hydrogenated at atmospheric pressure until one equivalent of hydrogen is taken up. Filtration and evaporation produces the product.

C. 1'-Phenyl spiro[(cyclohex-3-ene)-1,3'-[1,8]-naphthyridine]-2',4'-(1'H)-dione.

The cis-olefin (from part B) is dissolved/suspended in pyridine at 0°C. A slight excess of mesyl chloride (1.05 equivalents) is added and the mixture is stirred until reaction is complete (monitor by t.l.c.). The pyridine is removed under high vacuum and the residue is dissolved in $CH_2Cl_2$. The solution is washed with a small volume of cold water, dried, and treated with an excess (1.2 equivalents) of triethylamine. When reaction is complete the crude product is isolated by washing the $CH_2Cl_2$ with water, evaporation and chromatography in $CH_3CN:H_2O$ (80:20) over reversed-phase silica (Whatman Partisil (40;ODS—x3)), yields the product.

Example 3

1-Phenyl-3',4',5',6'-tetrahydro-spiro[1,8-naphthyridine-3,2'-(2H)pyran]-2,4-dione

A suspension of 4-Hydroxy-3-(4-hydroxybutyl)-1-phenyl-(1,8)-naphthyridin-2(1*H*)-one (2 g) in chloroform was stirred in an ice-bath. A solution of bromine (1 g) in chloroform was added dropwise and the mixture was stirred overnight at room temperature. To this mixture was added a solution of 1,8-diazabicyclo[5,4,0]undec-7-ene (2 g) in chloroform at room temperature. After about ¾ hr water was added and the pH was adjusted to be slightly acidic. The chloroform layer was separated and washed with saturated NaCl solution. The solution was dried and evaporated to a solid which was washed with ethanol/water and dried to yield 1-phenyl-3',4',5',6'-tetrahydro-spiro[1,8-naphthyridine-3,2'-(2*H*)pyran]-2,4-dione, m.p. 213—215°C.

Example 4

1-(3-Methoxyphenyl)-3',4',5',6'-tetrahydrospiro[1,8-naphthyridine-3,2'-(2H)pyran]-2,4-dione

To a suspension of 4-Hydroxy-3-(4-hydroxybutyl)-1-(3-methoxyphenyl)-(1,8)-naphthyridin-2(1*H*)-one (1 g) in methylene chloride (10 ml), in an ice-acetone bath was added a solution of bromine (0.5 g) in methylene chloride (5 ml) over a period of 15 min. The resulting yellow-orange suspension was stirred overnight at room temperature. To the resulting clear yellow solution was added a solution of 1,8-diazabicyclo[5,4,0]undec-7-ene (1 g), in methylene chloride (5 ml) during 5 mins. The mixture was stirred at room temperature for 3 hrs after which time water (5 ml) was added. The organic layer was separated, dried and evaporated. To the residue was added 50% aqueous ethanol (10 ml) and after some time the solid was filtered off and recrystallized from isopropanol to yield 1-(3-methoxyphenyl)-3',4',5',6'-tetrahydrospiro[1,8-naphthyridine-3,2'-(2*H*)pyran]-2,4-dione, m.p. 181—183°C.

By utilizing the appropriately substituted starting materials in the above-described procedure, the following products were obtained:

4,5-dihydro-1'-phenyl-spiro[furan-2(3*H*),3'2(*H*)-(1,8-naphthyridine]-2',4'-(1*H*)-dione, m.p. 241.5—243°C;

1-phenyl-spiro(1,8-naphthyridine-3,2'-oxetane)-2,4-dione, m.p. 233—235.5°C; and

1-(3-chlorophenyl)-3',4',5',6'-tetrahydrospiro[1,8-naphthyridine-3,2'(2*H*)-pyran]-2,4-dione, m.p. 158.5—160°C.

Example 5

1'-Ethoxycarbonyl-1-phenylspiro[1,8-naphthyridine 3,2'-piperidine]-2,4-dione

(A) N,2-Bis(ethoxycarbonyl)-2-(3-[2-chloronicotinoyl])-piperidine

A slight excess of 1M-lithium bis(trimethylsilyl)amide in tetrahydrofuran (THF) is cooled in a nitrogen atmosphere to below −60°C. To this is added a solution of ethyl N-ethoxycarbonyl pipecolinate (0.11M) in dry THF, dropwise.

Allow to stand for 2 hrs. Then add a solution of ethyl 2-chloronitocinate (0.1M) in dry THF, dropwise.

Allow to stand at −70°C for at least 4 hrs. Then warm gradually to room temperature. When no starting material remains, add acetic acid and water, and evaporate off the THF.

Isolate the product by extraction and purify by column chromatography.

(B) N-Ethoxycarbonyl-2-(3-[2-chloro-nicotinoyl)]-pipecolinoyl anilide

Carefully hydrolyze the product from part A using excess dilute NaOH in $H_2O$/EtOH. Follow by thin layer chromatography (TLC). When no starting material remains, adjust the pH to *ca* 9 with dil HCl and evaporate to low volume under reduced pressure. To the residue suspended in benzene, add an excess of oxalyl chloride, and warm the mixture until a reaction occurs.

When the reaction is complete evaporate off as much solvent and excess reagent as possible. Then add

aniline (2.2 equivs.) in dry THF. Warm to complete the reaction. Then isolate the product by adding water and acetic acid, evaporating off the THF and extracting into $CH_2Cl_2$. The product is purified by column chromatography.

(C) 1'-Ethoxycarbonyl-1-phenyl-spiro(1,8-naphthyridine-3,2'-piperidine)-2,4-dione

To a slight excess of 1M-lithium bis(trimethylsilyl)amide in THF, under nitrogen, at −70°C, is added the product from part B, in dry THF, dropwise. After standing for 2 hrs at −70°C the mixture is allowed to warm gradually in *ca* 20°C steps to room temperature. The reaction is followed by TLC at each step. When the reaction is complete, the product is isolated by addition of acetic acid and water. Removal of the THF, and extraction into $CH_2Cl_2$ is performed, and the product is purified by column chromatography.

Removal of the carbamate protecting group and subsequent modification of the resulting secondary amine are implemented by standard means well known to one skilled in the art.

Following the procedures exemplified above using the appropriate starting material and reaction conditions the following compounds may be prepared:

| Comp. No. | V | q | $Q^1$ | $Q^2$ | Y | d | m.p. °C | |
|---|---|---|---|---|---|---|---|---|
| 1 | O | 3 | Cl | Cl | O | N | | |
| 2 | O | 3 | H | Cl | O | N | 229—231.5 | hemihydrate |
| 3 | $CH_2$ | 2 | H | H | S | N | 188—189.5 | |
| 4 | $CH_2$ | 2 | —NH·CHO | H | O | N | 222—224 | |
| 5 | $CH_2$ | 2 | $—NH·CO·CO·OC_2H_5$ | H | O | N | 158—160 | |
| 6 | $CH_2$ | 2 | $—OCH_3$ | H | O | N | 159—160.5 | |
| 7 | $CH_2$ | 2 | —OH | H | O | | 218—220 | |
| 8 | $CH_2$ | 2 | $—NH_2$ | H | O | N | 200—202 | |
| 9 | $CH_2$ | 2 | $—OCH_2CO·O·C_2H_5$ | H | O | N | 103—105 | |
| 10 | $CH_2$ | 2 | H | H | O | CH | | |
| 11 | O | 3 | H | $—OCH_3$ | O | N | | hemihydrobromide |

**Claims for the Contracting States: BE CH DE FR IT LI LU NL SE**

1. Compounds of the general formula I

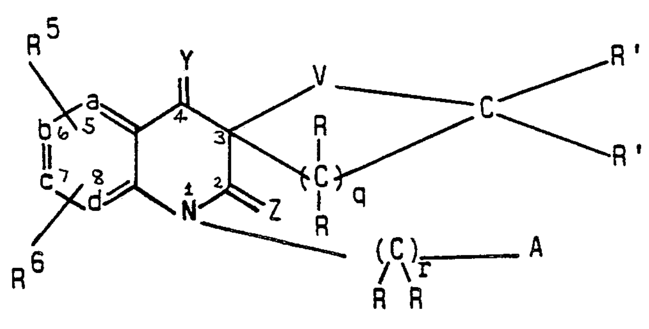

I

wherein

two of the ring atoms a, b, c and d may be CH or N and the remaining two atoms represent CH;

Y and Z independently represent O or S;

V represents O, $S(O)_n$, $N—R^8$ or

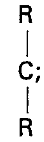

each R independently represents hydrogen, $C_1$ to $C_6$ alkyl, $CH_2OH$, $COR_7$ (wherein $R^7$ represents hydrogen or $C_1$ to $C_6$ alkyl) or hydroxy, with the proviso that only one hydroxy group can be attached to one carbon atom;

each R′ independently is as defined for R above, except that when V represents O, $S(O)_n$ or N—$R^8$, R′ may not be hydroxy;

$R^5$ and $R^6$ may be the same or different and are hydrogen, alkyl having from 1 to 6 carbon atoms, halogen, nitro, alkoxy having from 1 to 6 carbon atoms, trifluoromethyl, alkylthio having 1 to 6 carbon atoms or cyano; $R^8$ is hydrogen, alkyl having from 1 to 6 carbon atoms, carboxylic acyl having from 2 to 7 carbon atoms, alkylsulfonyl having from 1 to 6 carbon atoms, carboalkoxy having from 2 to 7 carbon atoms, $CONH_2$, phenyl or pyridyl of which the last two may be substituted with up to three substituents, Q, whereby each Q independently is hydroxy, alkyl having from 1 to 6 carbon atoms, halogen, nitro, alkoxy having from 1 to 6 carbon atoms, trifluoromethyl, cyano, cycloalkyl, having from 3 to 7 carbon atoms, alkenyloxy having from 3 to 6 carbon atoms, alkynyloxy having from 3 to 6 carbon atoms, $S(O)_n$—$R^a$ [wherein n is defined herein and $R^a$ is alkyl having from 1 to 6 carbon atoms], $NHSO_2R^a$ [wherein $R^a$ is defined herein], $NHSO_2CF_3$, $SO_2NH_2$, $COR^b$ [wherein $R^b$ is OH, $NH_2$ or $OR^a$ (wherein $R^a$ is defined herein)], O—B—$COR^a$ [wherein B is alkylene having from 1 to 4 carbon atoms and $R^b$ is defined herein], or $NHCOR^c$ [wherein $R^c$ is hydrogen, alkyl having from 1 to 6 carbon atoms, alkoxy having from 1 to 6 carbon atoms, $COR^d$ (wherein $R^d$ is hydroxy or alkoxy having from 1 to 6 carbon atoms) or $NHR^e$ (wherein $R^e$ is hydrogen or alkyl having 1 to 6 carbon atoms)];

n is 0, 1 or 2;

r is 0, 1 or 2;

q is an integer of from 1 to 5; and

A is phenyl, naphthyl, indenyl, indanyl, pyridyl, pyrimidyl, pyrazinyl, furyl, thienyl, imidazolyl, thiazolyl or oxazolyl, any of which may be substituted with up to three substituents Q as defined herein above, and salts and solvates of such compounds.

2. Compounds of claim 1 wherein Y and Z are both oxygen.

3. Compounds of claims 1 or 2 wherein r is zero.

4. Compounds of claims 1 to 3 wherein $R^5$ and $R^6$ are both hydrogen.

5. Compounds of claims 1 to 4 wherein all of a, b, c, and d are CH.

6. Compounds of claims 1 to 4 wherein a and d are both N and b and c are both CH.

7. Compounds of claims 1 to 4 wherein d is N and a, b and c are CH.

8. Compounds of claims 1 to 7 wherein q is 2, 3 or 4.

9. Compounds of claims 1 to 8 wherein R and R′ independently are hydrogen, methyl, ethyl, propyl, isopropyl, n-butyl or iso-butyl.

10. Compounds of claims 1 to 9 wherein A is phenyl or phenyl substituted with one or two substituents Q as defined in claim 1.

11. Compounds of claims 1 to 10 wherein V is O, $S(O)_n$ or N—$R^8$.

12. Compounds of claim 11 wherein V is O.

13. The compounds:

1′-phenyl-spiro[cyclopentane-1,3′-(1,8)naphthyridine]-2′,4′-(1′*H*)-dione;
1′-phenyl-spiro[cyclopentane-1,3′-quinoline]-2′,4′-(1′*H*)-dione;
1′-(4-methylphenyl)spiro[cyclopentane-1,3′-(1,8)naphthyridine]-2′,4′-(1′*H*)-dione;
1′-(4-chlorophenyl)spiro[cyclopentane-1,3′-(1,8)naphthyridine]-2′,4′-(1′*H*)-dione;
1′-(3,4-dichlorophenyl)spiro[cyclopentane-1,3′-(1,8)naphthyridine]-2′,4′-(1′*H*)-dione;
1′-(3-chlorophenyl)spiro[cyclopentane-1,3′-(1,8)naphthyridine]-2′,4′-(1′*H*)-dione;
1′-(3-methoxyphenyl)spiro[cyclopentane-1,3′-(1,8)naphthyridine]-2′,4′-(1′*H*)-dione;
1′-(3-hydroxyphenyl)spiro[cyclopentane-1,3′-(1,8)naphthyridine]-2′,4′-(1′*H*)-dione.

14. The compounds:

1-phenyl-3′,4′,5′,6′-tetrahydrospiro[1,8-naphthyridine-3,2′-(2*H*)pyran]-2,4-dione;
1-(3-methoxyphenyl)-3′,4′,5′,6′-tetrahydrospiro[1,8-naphthyridine-3,2′-(2*H*)pyran]-2,4-dione;
4,5-dihydro-1′-phenyl-spiro[furan-2(3*H*),3′(2′*H*)(1,8)naphthyridine]-2′,4′(1′*H*)-dione;
1-phenyl-spiro[1,8-naphthyridine-3,2′-oxetane]-2,4-dione; and
1-(3-chlorophenyl)-3′,4′,5′,6′-tetrahydrospiro[1,8-naphthyridine-3,2′-(2*H*)pyran]-2,4-dione.

15. Pharmaceutical compositions comprising as an active ingredient a compound as defined in any one of claims 1 to 14 in admixture with a suitable pharmaceutical carrier.

16. Process for preparing pharmaceutical compositions as defined in claim 15, which comprises mixing a compound as defined in any one of claims 1 to 14 with a suitable pharmaceutical carrier.

17. Process for the preparation of compounds of the general formula I as defined in claim 1, characterized in that a compound of the general formula IV

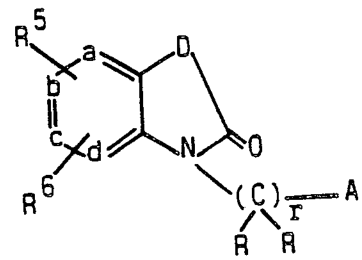

IV

wherein a, b, c, d, R, $R^5$, $R^6$, A and r are as defined above and D represents either of the groups

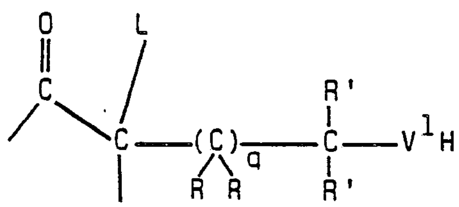

or

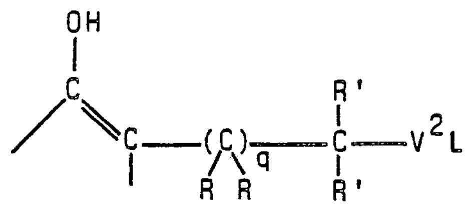

VI VII

wherein L is a good leaving group, $V^1$ is O, $S(O)_n$ or

N—$R^8$, $V^2$ is 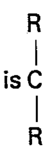

and R, R′ and q are as defined above, is subjected to an intramolecular condensation yielding a compound of formula I wherein both Y and Z are oxygen, followed, if desired, by replacing one or both of the oxygen atoms represented by Y and Z with sulfur.

**Claims for the Contracting State: AT**

1. Process for the preparation of compounds of the general formula I

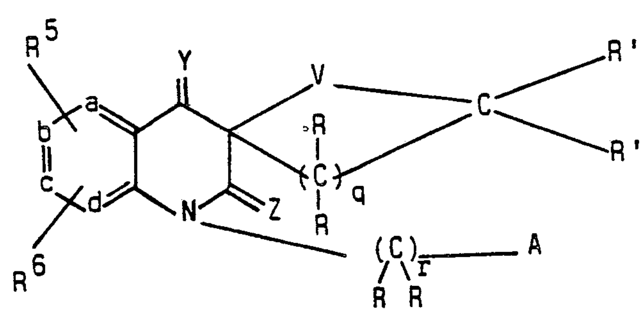

I

wherein

two of the ring atoms a, b, c and d may be CH or N and the remaining two atoms represent CH;

Y and Z independently represent O or S;

V represents O, $S(O)_n$, N—$R^8$ or

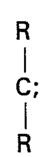

each R independently represents hydrogen, $C_1$ to $C_6$ alkyl, $CH_2OH$, $COR_7$ (wherein $R^7$ represents hydrogen or $C_1$ to $C_6$ alkyl) or hydroxy, with the proviso that only one hydroxy group can be attached to one carbon atom;

each R′ independently is as defined for R above, except that when V represents O, $S(O)_n$ or N—$R^8$, R′ may not be hydroxy;

$R^5$ and $R^6$ may be the same or different and are hydrogen, alkyl having from 1 to 6 carbon atoms, halogen, nitro, alkoxy having from 1 to 6 carbon atoms, trifluoromethyl, alkylthio having 1 to 6 carbon atoms or cyano; $R^8$ is hydrogen, alkyl having from 1 to 6 carbon atoms, carboxylic acyl having from 2 to 7 carbon atoms, alkylsulfonyl having from 1 to 6 carbon atoms, carboalkoxy having from 2 to 7 carbon atoms, $CONH_2$, phenyl or pyridyl of which the last two may be substituted with up to three substituents, Q, whereby each Q independently is hydroxy, alkyl having from 1 to 6 carbon atoms, halogen, nitro, alkoxy having from 1 to 6 carbon atoms, trifluoromethyl, cyano, cycloalkyl, having from 3 to 7 carbon atoms, alkenyloxy having from 3 to 6 carbon atoms, alkynyloxy having from 3 to 6 carbon atoms, $S(O)_n$—$R^a$ [wherein n is defined herein and $R^a$ is alkyl having from 1 to 6 carbon atoms], $NHSO_2R^a$ [wherein $R^a$ is defined herein], $NHSO_2CF_3$, $SO_2NH_2$, $COR^b$ [wherein $R^b$ is OH, $NH_2$ or $OR^a$ (wherein $R^a$ is defined herein)], O—B—$COR^a$ [wherein B is alkylene having from 1 to 4 carbon atoms and $R^b$ is defined herein], or $NHCOR^c$ [wherein $R^c$ is hydrogen, alkyl having from 1 to 6 carbon atoms, alkoxy having from 1 to 6 carbon atoms, $COR^d$ (wherein $R^d$ is hydroxy or alkoxy having from 1 to 6 carbon atoms) or $NHR^e$ (wherein $R^e$ is hydrogen or alkyl having 1 to 6 carbon atoms)];

n is 0, 1 or 2;

r is 0, 1 or 2;

q is an integer of from 1 to 5; and

A is phenyl, naphthyl, indenyl, indenyl, pyridyl, pyrimidyl, pyrazinyl, furyl, thienyl, imidazolyl, thiazolyl or oxazolyl, any of which may be substituted with up to three substituents Q as defined herein above, and salts and solvates of such compounds, characterized in that a compound of the general formula

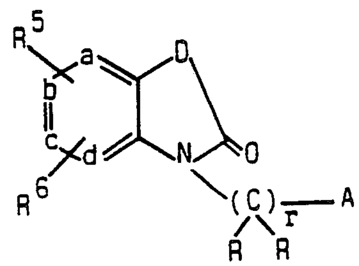

IV

wherein a, b, c, d, R, $R^5$, $R^6$, A and r are as defined above and D represents either of the groups

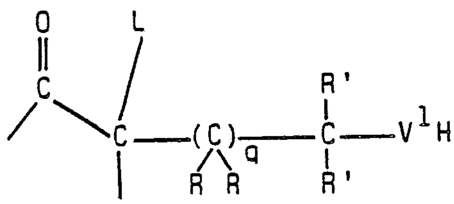

VI

or

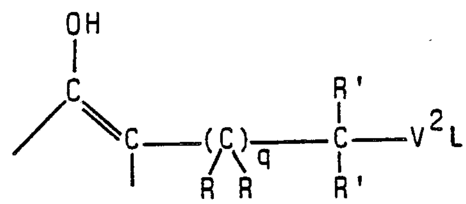

VII

wherein L is a good leaving group, $V^1$ is O, $S(O)_n$ or

N—$R^8$, $V^2$ is 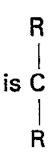

and R, R′ and q are as defined above, is subjected to an intramolecular condensation yielding a compound of formula I wherein both Y and Z are oxygen, followed, if desired, by replacing one or both of the oxygen atoms represented by Y and Z with sulfur.

2. Process according to claim 1, characterized in that the intramolecular condensation is accomplished by treating the compound of formula IV in an inert solvent with a base.

3. Process according to claim 2, characterized in that an organic base is used.

4. Process according to any one of claims 1 to 3, characterized in that the conversion of oxygen atoms

represented by X and/or Y into sulfur is accomplished by treatment with an appropriate sulfide.

5. Process according to any one of claims 1 to 4, characterized in that the leaving group L is selected from bromide, iodide, trifluoroacetoxy, p-toluenesulfonyloxy and methanesulfonyloxy.

6. Process according to any one of claims 1 to 5, characterized in that compounds of formula I wherein Y and Z are both oxygen, r is zero, a, b and c are CH and d is N or CH, q is 2, 3 or 4 and A is phenyl or phenyl substituted by one or two substituents Q [Q being as defined in claim 1] are prepared.

7. Process according to any one of claims 1 to 6, characterized in that the compounds
1′-phenyl-spiro[cyclopentane-1,3′-(1,8)naphthyridine]-2′,4′-(1′*H*)-dione;
1′-phenyl-spiro[cyclopentane-1,3′-quinoline]-2′,4′-(1′*H*)-dione;
1′-(4-methylphenyl)spiro[cyclopentane-1,3′-(1,8)naphthyridine]-2′,4′-(1′*H*)-dione;
1′-(4-chlorophenyl)spiro[cyclopentane-1,3′-(1,8)naphthyridine]-2′,4′-(1′*H*)-dione;
1′-(3,4-dichlorophenyl)spiro[cyclopentane-1,3′-(1,8)naphthyridine]-2′,4′-(1′*H*)-dione;
1′-(3-chlorophenyl)spiro[cyclopentane-1,3′-(1,8)naphthyridine]-2′,4′-(1′*H*)-dione;
1′-(3-methoxyphenyl)spiro[cyclopentane-1,3′-(1,8)naphthyridine]-2′,4′-(1′*H*)-dione;
1′-(3-hydroxyphenyl)spiro[cyclopentane-1,3′-(1,8)naphthyridine]-2′,4′-(1′*H*)-dione.
1-phenyl-3′,4′,5′,6′-tetrahydro-spiro[1,8-naphthyridine-3,2′-(2*H*)pyran]-2,4-dione;
1-(3-methoxyphenyl)-3′,4′,5′,6′-tetrahydro-spiro[1,8-naphthyridine-3,2′-(2*H*)pyran]-2,4-dione;
4,5-dihydro-1′-phenyl-spiro[furan-2(3*H*),3′(2′*H*)(1,8)naphthyridine]-2′,4′(1′*H*)-dione;
1-phenyl-spiro[1,8-naphthyridine-3,2′-oxetane]-2,4-dione; and
1-(3-chlorophenyl)-3′,4′,5′,6′-tetrahydro-spiro[1,8-naphthyridine-3,2′-(2*H*)pyran]-2,4-dione are prepared.

8. Process for preparing pharmaceutical compositions, characterized in that a compound as defined in any one of claims 1 to 7 is mixed with a suitable pharmaceutical carrier.

**Patentansprüche für die Vertragsstaaten: BE CH DE FR IT LI LU NL SE**

1. Verbindungen der allgemeinen Formel I

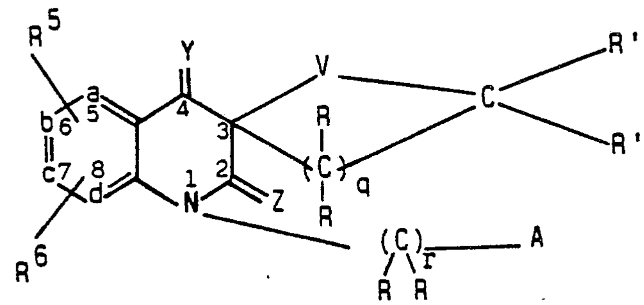

I

in der

zwei der Ring-Atome a, b, c und d CH oder N sein können und die verbleibenden zwei Atome CH darstellen;

Y und Z unabhängig voneinander O oder S darstellen;

V O, $S(O)_n$, N—$R^8$ oder

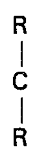

darstellt;

R jeweils unabhängig Wasserstoff, $C_1$- bis $C_6$-Alkyl, $CH_2OH$, $COR^7$ (worin $R^7$ Wasserstoff oder $C_1$- bis $C_6$-Alkyl darstellt) oder Hydroxy darstellt, mit der Maßgabe, daß nur eine Hydroxy-Gruppe an einem Kohlenstoff-Atom haften kann;

R′ jeweils unabhängig die im Vorstehenden für R angegebenen Bedeutungen hat, mit der Ausnahme, daß dann, wenn V O, $S(O)_n$ oder N—$R^8$ darstellt, R′ nicht Hydroxy sein kann;

$R^5$ und $R^6$ gleich oder verschieden sein können und Wasserstoff, Alkyl mit 1 bis 6 Kohlenstoff-Atomen, Halogen, Nitro, Alkoxy mit 1 bis 6 Kohlenstoff-Atomen, Trifluoromethyl, Alkylthio mit 1 bis 6 Kohlenstoff-Atomen oder Cyano sind;

$R^8$ Wasserstoff, Alkyl mit 1 bis 6 Kohlenstoff-Atomen, carbocyclisches Acyl mit 2 bis 7 Kohlenstoff-Atomen, Alkylsulfonyl mit 1 bis 6 Kohlenstoff-Atomen, Carboalkoxy mit 2 bis 7 Kohlenstoff-Atomen, $CONH_2$, Phenyl oder Pyridyl, von denen die beiden letzten mit bis zu drei Substituenten Q substituiert sein können, wobei jedes Q unabhängig Wasserstoff, Alkyl mit 1 bis 6 Kohlenstoff-Atomen, Halogen, Nitro, Alkoxy mit 1 bis 6 Kohlenstoff-Atomen, Trifluoromethyl, Cyano, Cycloalkyl mit 3 bis 7 Kohlenstoff-Atomen,

Alkenyloxy mit 3 bis 6 Kohlenstoff-Atomen, Alkinyloxy mit 3 bis 6 Kohlenstoff-Atomen, $S(O)_n$—$R^a$ [worin n hierin definiert wird und $R^a$ Alkyl mit 1 bis 6 Kohlenstoff-Atomen ist], $NHSO_2R^a$ [worin $R^a$ die angegebene Bedeutung hat], $NHSO_2CF_3$, $SO_2NH_2$, $COR^b$ [worin $R^b$ OH, $NH_2$ oder $OR^a$ ist (worin $R^a$ die angegebene Bedeutung hat)], O—B—$COR^b$ [worin B Alkylen mit 1 bis 4 Kohlenstoff-Atomen ist und $R^b$ die angegebene Bedeutung hat] oder $NHCOR^c$ [worin $R^c$ Wasserstoff, Alkyl mit 1 bis 6 Kohlenstoff-Atomen, Alkoxy mit 1 bis 6 Kohlenstoff-Atomen, $COR^d$ (worin $R^d$ Hydroxy oder Alkoxy mit 1 bis 6 Kohlenstoff-Atomen ist) oder $NHR^e$ ist (worin $R^e$ Wasserstoff oder Alkyl mit 1 bis 6 Kohlenstoff-Atomen ist)] ist

n 0, 1 oder 2 ist

r 0, 1 oder 2 ist

q eine ganze Zahl von 1 bis 5 ist und

A Phenyl, Naphthyl, Indenyl, Indanyl, Pyridyl, Pyrimidyl, Pyrazinyl, Furyl, Thienyl, Imidazolyl, Thiazolyl oder Oxazolyl ist, von denen jedes mit bis zu drei Substituenten Q, wie sie im Vorstehenden definiert sind, substituiert sein kann,

und Salze und Solvate solcher Verbindungen.

2. Verbindungen nach Anspruch 1, worin Y und Z beide Sauerstoff sind.

3. Verbindungen nach Anspruch 1 oder 2, worin r Null ist.

4. Verbindungen nach Anspruch 1 bis 3, worin $R^5$ und $R^6$ beide Wasserstoff sind.

5. Verbindungen nach Anspruch 1 bis 4, worin a, b, c und d sämtlich CH sind.

6. Verbindungen nach Anspruch 1 bis 4, worin a und d beide N sind und b und c beide CH sind.

7. Verbindungen nach Anspruch 1 bis 4, worin d N ist und a, b und c CH sind.

8. Verbindungen nach Anspruch 1 bis 7, worin q 2, 3 oder 4 ist.

9. Verbindungen nach Anspruch 1 bis 8, worin R und R′ unabhängig Wasserstoff, Methyl, Ethyl, Propyl, iso-Propyl, n-Butyl oder iso-Butyl sind.

10. Verbindungen nach Anspruch 1 bis 9, worin A Phenyl oder mit ein oder zwei Substituenten Q, wie sie in Anspruch 1 definiert sind, substituiertes Phenyl ist.

11. Verbindungen nach Anspruch 1 bis 10, worin V O, $S(O)_n$ oder N—$R^8$ ist.

12. Verbindungen nach Anspruch 11, worin V O ist.

13. Die Verbindungen:

1′-Phenyl-spiro[cyclopentan-1,3′-(1,8)naphthyridin]-2′,4′-(1′*H*)-dion;

1′-Phenyl-spiro[cyclopentan-1,3′-chinolin]-2′,4′-(1′*H*)-dion;

1′-(4-Methylphenyl)spiro[cyclopentan-1,3′-(1,8)naphthyridin]-2′,4′-(1′*H*)-dion;

1′-(4-Chlorophenyl)spiro[cyclopentan-1,3′-(1,8)naphthyridin]-2′,4′-(1′*H*)-dion;

1′-(3,4-Dichlorophenyl)spiro[cyclopentan-1,3′-(1,8)naphthyridin]-2′,4′-(1′*H*)-dion;

1′-(3-Chlorophenyl)spiro[cyclopentan-1,3′-(1,8)naphthyridin]-2′,4′-(1′*H*)-dion;

1′-(3-Methoxyphenyl)spiro[cyclopentan-1,3′-(1,8)naphthyridin]-2′,4′-(1′*H*)-dion;

1′-(3-Hydroxyphenyl)spiro[cyclopentan-1,3′-(1,8)naphthyridin]-2′,4′-(1′*H*)-dion;

1′-Phenylspiro[cyclopentan-1,3′-chinolin]-2′,4′-(1′*H*)-dion.

14. Die Verbindungen:

1-Phenyl-3′,4′,5′,6′-tetrahydrospiro[1,8-naphthyridin-3,2′-(2*H*)pyran]-2,4-dion;

1-(3-Methoxyphenyl)-3′,4′,5′,6′-tetrahydrospiro[1,8-naphthyridin-3,2′-(2*H*)pyran]-2,4-dion;

4,5-Dihydro-1′-phenyl-spiro[furan-2(3*H*),3′(2′*H*)-(1,8)naphthyridin]-2′,4′-(1′*H*)-dion;

1-Phenyl-spiro[1,8-naphthyridin-3,2′-oxetan]-2,4-dion und

1-(3-Chlorophenyl)-3′,4′,5′,6′-tetrahydrospiro[1,8-naphthyridin-3,2′-(2*H*)pyran]-2,4-dion.

15. Pharmazeutische Zusammensetzungen, umfassend als aktiven Bestandteil eine Verbindung nach irgendeinem der Ansprüche 1 bis 14 im Gemisch mit einem geeigneten pharmazeutischen Träger.

16. Verfahren zur Herstellung pharmazeutischer Zusammensetzungen nach Anspruch 15, umfassend das Vermischen einer Verbindung nach irgendeinem der Ansprüche 1 bis 14 mit einem geeigneten pharmazeutischen Träger.

17. Verfahren zur Herstellung von Verbindungen der allgemeinen Formel I nach Anspruch 1, dadurch gekennzeichnet, daß eine Verbindung der allgemeinen Formel IV

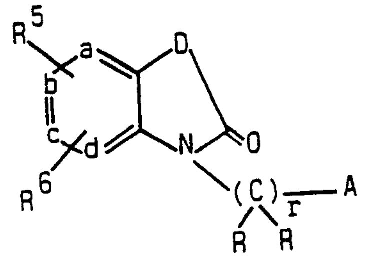

IV

in der a, b, c, d, R, $R^5$, $R^6$, A und r die im Vorstehenden angegebenen Bedeutungen haben und D eine der Gruppen

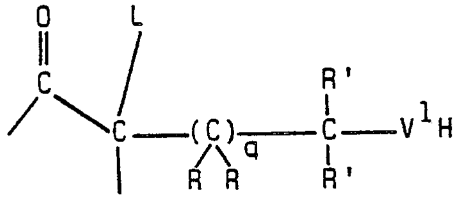

oder

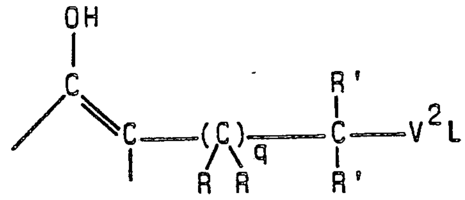

VI VII

darstellt, in denen

L eine gut abspaltbare Gruppe ist,

$V^1$ O, $S(O)_n$ oder $N—R^8$ ist,

$V^2$

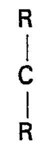

ist und

R, R′ und q die im Vorstehenden angegebenen Bedeutungen haben,

einer intromolekularen Kondensation unterworfen wird, die eine Verbindung der Formel I ergibt, in der sowohl Y als auch Z Sauerstoff sind, und anschließend gewünschtenfalls ein oder beide der durch Y und Z dargestellten Sauerstoff-Atome durch Schwefel ersetzt werden.

**Patentansprüche für den Vertragsstaat: AT**

1. Verfahren zur Herstellung von Verbindungen der allgemeinen Formel I

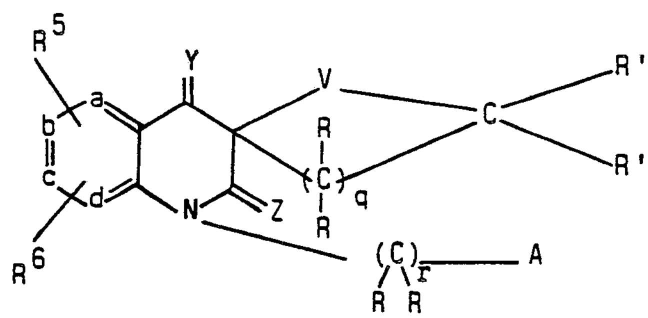

I

in der

zwei der Ring-Atome a, b, c und d CH oder N sein können und die verbleibenden zwei Atome CH darstellen;

Y und Z unabhängig voneinander O oder S darstellen;

V O, $S(O)_n$, $N—R^8$ oder

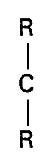

darstellt;

R jeweils unabhängig Wasserstoff, $C_1$- bis $C_6$-Alkyl, $CH_2OH$, $COR^7$ (worin $R^7$ Wasserstoff oder $C_1$- bis $C_6$-Alkyl darstellt) oder Hydroxy darstellt, mit der Maßgabe, daß nur eine Hydroxy-Gruppe an einem Kohlenstoff-Atom haften kann;

R′ jeweils unabhängig die im Vorstehenden für R angegebenen Bedeutungen hat, mit der Ausnahme, daß dann, wenn V O, $S(O)_n$ oder $N—R^8$ darstellt, R′ nicht Hydroxy sein kann;

$R^5$ und $R^6$ gleich oder verschieden sein können und Wasserstoff, Alkyl mit 1 bis 6 Kohlenstoff-Atomen, Halogen, Nitro, Alkoxy mit 1 bis 6 Kohlenstoff-Atomen, Trifluoromethyl, Alkylthio mit 1 bis 6 Kohlenstoff-Atomen oder Cyano sind;

$R^8$ Wasserstoff, Alkyl mit 1 bis 6 Kohlenstoff-Atomen, carbocyclisches Acyl mit 2 bis 7 Kohlenstoff-Atomen, Alkylsulfonyl mit 1 bis 6 Kohlenstoff-Atomen, Carboalkoxy mit 2 bis 7 Kohlenstoff-Atomen,

$CONH_2$, Phenyl oder Pyridyl, von denen die beiden letzten mit bis zu drei Substituenten Q substituiert sein können, wobei jedes Q unabhängig Wasserstoff, Alkyl mit 1 bis 6 Kohlenstoff-Atomen, Halogen, Nitro, Alkoxy mit 1 bis 6 Kohlenstoff-Atomen, Trifluoromethyl, Cyano, Cycloalkyl mit 3 bis 7 Kohlenstoff-Atomen, Alkenyloxy mit 3 bis 6 Kohlenstoff-Atomen, Alkinyloxy mit 3 bis 6 Kohlenstoff-Atomen, $S(O)_n—R^a$ [worin n hierin definiert wird und $R^a$ Alkyl mit 1 bis 6 Kohlenstoff-Atomen ist], $NHSO_2R^a$ [worin $R^a$ die angegebene Bedeutung hat], $NHSO_2CF_3$, $SO_2NH_2$, $COR^b$ [worin $R^b$ OH, $NH_2$ oder $OR^a$ ist (worin $R^a$ die angegebene Bedeutung hat)], $O—B—COR^b$ [worin B Alkylen mit 1 bis 4 Kohlenstoff-Atomen ist und $R^b$ die angegebene Bedeutung hat] oder $NHCOR^c$ [worin $R^c$ Wasserstoff, Alkyl mit 1 bis 6 Kohlenstoff-Atomen, Alkoxy mit 1 bis 6 Kohlenstoff-Atomen, $COR^d$ (worin $R^d$ Hydroxy oder Alkoxy mit 1 bis 6 Kohlenstoff-Atomen ist) oder $NHR^e$ ist (worin $R^e$ Wasserstoff oder Alkyl mit 1 bis 6 Kohlenstoff-Atomen ist)] ist

n 0, 1 oder 2 ist

r 0, 1 oder 2 ist

q eine ganze Zahl von 1 bis 5 ist und

A Phenyl, Naphthyl, Indenyl, Indanyl, Pyridyl, Pyrimidyl, Pyrazinyl, Furyl, Thienyl, Imidazolyl, Thiazolyl oder Oxazolyl ist, von denen jedes mit bis zu drei Substituenten Q, wie sie im Vorstehenden definiert sind, substituiert sein kann,

und von Salzen und Solvaten solcher Verbindungen, dadurch gekennzeichnet, daß eine Verbindung der allgemeinen Formel IV

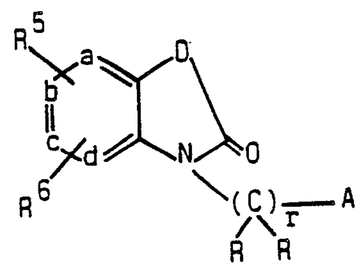

IV

in der a, b, c, d, R, $R^5$, $R^6$, A und r die im Vorstehenden angegebenen Bedeutungen haben und D eine der Gruppen

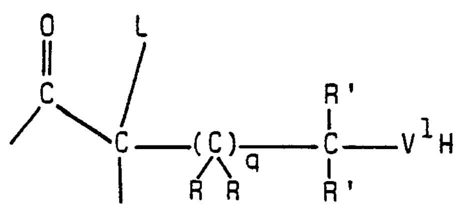

VI

oder

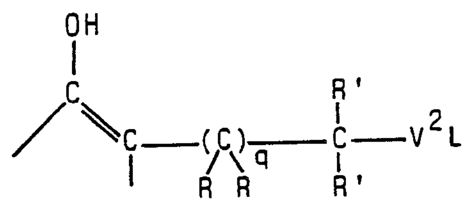

VII

darstellt, in denen

L eine gut abspaltbare Gruppe ist,

$V^1$ O, $S(O)_n$ oder $N—R^8$ ist,

$V^2$

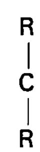

ist und

R, R' und q die im Vorstehenden angegebenen Bedeutungen haben,

einer intromolekularen Kondensation unterworfen wird, die eine Verbindung der Formel I ergibt, in der sowohl Y als auch Z Sauerstoff sind, und anschließend gewünschtenfalls ein oder beide der durch Y und Z dargestellten Sauerstoff-Atome durch Schwefel ersetzt werden.

2. Verfahren nach Anspruch 1, dadurch gekennzeichnet, daß die intramolekulare Kondensation durch Behandeln der Verbindung der Formel IV mit einer Base in einem inerten Lösungsmittel vorgenommen wird.

3. Verfahren nach Anspruch 2, dadurch gekennzeichnet, daß eine organische Base verwendet wird.

4. Verfahren nach irgendeinem der Ansprüche 1 bis 3, dadurch gekennzeichnet, daß der Ersatz der durch X und/oder Y dargestellten Sauerstoff-Atome durch Behandlung mit einem geeigneten Sulfid vorgenommen wird.

5. Verfahren nach irgendeinem der Ansprüche 1 bis 4, dadurch gekennzeichnet, daß die abspaltbare Gruppe L aus Bromid, Iodid, Trifluoroacetoxy, p-Toluolsulfonyloxy und Methansulfonyloxy ausgewählt ist.

6. Verfahren nach irgendeinem der Ansprüche 1 bis 5, dadurch gekennzeichnet, daß Verbindungen der Formel I hergestellt werden, in denen

Y und Z beide Sauerstoff sind,

r Null ist,

a, b und c CH sind und d N oder CH ist,

q 2, 3 oder 4 ist und

A Phenyl oder mit ein oder zwei Substituenten Q [wobei Q wie in Anspruch 1 definiert ist] substituiertes Phenyl ist,

hergestellt werden.

7. Verfahren nach irgendeinem der Ansprüche 1 bis 6, dadurch gekennzeichnet, daß die Verbindungen

1'-Phenyl-spiro[cyclopentan-1,3'-(1,8)naphthyridin]-2',4'-(1'*H*)-dion;

1'-Phenyl-spiro[cyclopentan-1,3'-chinolin]-2',4'-(1'*H*)-dion;

1'-(4-Methylphenyl)spiro[cyclopentan-1,3'-(1,8)naphthyridin]-2',4'-(1'*H*)-dion;

1'-(4-Chlorophenyl)spiro[cyclopentan-1,3'-(1,8)naphthyridin]-2',4'-(1'*H*)-dion;

1'-(3,4-Dichlorophenyl)spiro[cyclopentan-1,3'-(1,8)naphthyridin]-2',4'-(1'*H*)-dion;

1'-(3-Chlorophenyl)spiro[cyclopentan-1,3'-(1,8)naphthyridin]-2',4'-(1'*H*)-dion;

1'-(3-Methoxyphenyl)spiro[cyclopentan-1,3'-(1,8)naphthyridin]-2',4'-(1'*H*)-dion;

1'-(3-Hydroxyphenyl)spiro[cyclopentan-1,3'-(1,8)naphthyridin]-2',4'-(1'*H*)-dion;

1'-Phenylspiro[cyclopentan-1,3'-chinolin]-2',4'-(1'*H*)-dion;

1-Phenyl-3',4',5',6'-tetrahydrospiro[1,8-naphthyridin-3,2'-(2*H*)pyran]-2,4-dion;

1-(3-Methoxyphenyl)-3',4',5',6'-tetrahydrospiro[1,8-naphthyridin-3,2'-(2*H*)pyran]-2,4-dion;

4,5-Dihydro-1'-phenyl-spiro[furan-2(3*H*),3'(2'*H*)-(1,8)naphthyridin]-2',4'-(1'*H*)-dion;

1-Phenyl-spiro[1,8-naphthyridin-3,2'-oxetan]-2,4-dion und

1-(3-Chlorophenyl)-3',4',5',6'-tetrahydrospiro[1,8-naphthyridin-3,2'-(2*H*)pyran]-2,4-dion

hergestellt werden.

8. Verfahren zur Herstellung pharmazeutischer Zusammensetzungen dadurch gekennzeichnet, daß eine Verbindung nach irgendeinem der Ansprüche 1 bis 7 mit einem geeigneten pharmazeutischen Träger vermischt wird.

**Revendications pour les Etats contractants: BE CH DE FR IT LI LU NL SE**

1. Composés de formule générale I:

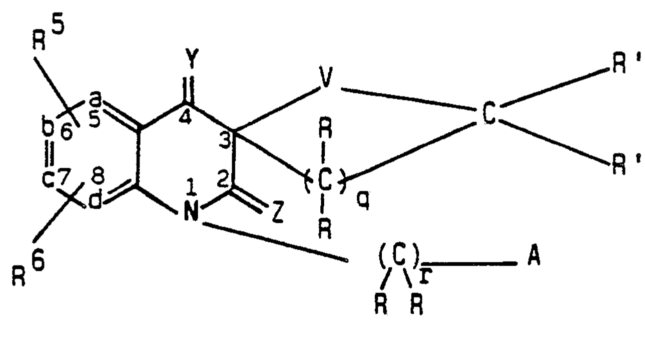

I

dans laquelle

deux des atomes a, b, c et d du noyau peuvent être CH ou N et les deux atomes restants représentent CH;

Y et Z représentent indépendamment O ou S;

V représente O, $S(O)_n$, $N—R^8$ ou

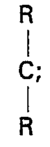

chaque R représente indépendamment un hydrogène, un alkyle de $C_1$ à $C_6$, $CH_2OH$, $COR_7$ (où $R^7$ représente un hydrogène ou un alkyle de $C_1$ à $C_6$) ou un hydroxy, à la condition que uniquement un groupement hydroxy soit attaché à un atome de carbone;

chaque R' indépendamment est défini comme pour R ci-dessus, excepté que lorsque V représente O, $S(O)_n$ ou $N—R^8$, R' ne peut pas être hydroxy;

$R^5$ et $R^6$ peuvent être les mêmes ou différents et sont hydrogène, alkyle ayant de 1 à 6 atomes de carbone, halogène, nitro, alkoxy ayant de 1 à 6 atomes de carbone, trifluorométhyle, alkylthio ayant de 1 à 6

atomes de carbone ou cyano; $R^8$ est hydrogène, alkyle ayant de 1 à 6 atomes de carbone, alcyle carboxylique ayant de 2 à 7 atomes de carbone, alkylsulfonyle ayant de 1 à 6 atomes de carbone, carboalkoxy ayant de 2 à 7 atomes de carbone, $CONH_2$, phényle ou pyridyle dont les deux derniers peuvent être substitués avec jusqu'à trois substituants, Q, où chaque Q est indépendamment hydroxy, alkyle ayant de 1 à 6 atomes de carbone, halogène, nitro, alcoxy ayant de 1 à 6 atomes de carbone, trifluorométhyle, cyano, cycloalkyle ayant de 3 à 7 atomes de carbone, alcényloxy ayant de 3 à 6 atomes de carbone, alkynyloxy ayant de 3 à 6 atomes de carbone, $S(O)_n—R^a$ [où n est défini ci-dessous et $R^a$ est un alkyle ayant de 1 à 6 atomes de carbone], $NHSO_2R^a$ [où $R^a$ est défini ci-dessous], $NHSO_2CF_3$, $SO_2NH_2$, $COR^b$ [où $R^b$ est OH, $NH_2$ ou $OR^a$ (où $R^a$ est défini ci-dessous)], $O—B—COR^b$ [où B est un alkylène ayant de 1 à 4 atomes de carbone et $R^b$ est défini ci-dessous], ou $NHCOR^c$ [où $R^c$ est un hydrogène, un alkyle ayant de 1 à 6 atomes de carbone, un alkoxy ayant de 1 à 6 atomes de carbone, $COR^d$ (où $R^d$ est hydroxy ou alcoxy ayant de 1 à 6 atomes de carbone) ou $NHR^e$ (où $R^e$ est hydrogène ou alkyle ayant de 1 à 6 atomes de carbones)];

n est 0, 1 ou 2;

r est 0, 1 ou 2;

q est un nombre entier de 1 à 5; et

A est phényle, naphtyle, indényle, indanyle, pyridyle, pyrimidyle, pyrazinyle, furyle, thiényle, imidazolyle, thiazolyle, ou oxazolyle, dont l'un quelconque peut être substitué avec jusqu'à trois substituants Q tels que définis ci-dessus, et les sels et les solvates de tels composés.

2. Composés selon la revendication 1 où Y et Z sont tous les deux oxygène.

3. Composés selon les revendications 1 ou 2 où r est égal à zéro.

4. Composés selon les revendications 1 à 3 où $R^1$ et $R^6$ sont tous les deux hydrogène.

5. Composés selon les revendications 1 à 4 où tous les a, b, c et d sont CH.

6. Composés selon les revendications 1 à 4 où a et d sont tous les deux N et b et c sont tous les deux CH.

7. Composés selon les revendications 1 à 4 où d est N et a, b, c sont CH.

8. Composés selon les revendications 1 à 7 où q est 2, 3 ou 4.

9. Composés selon les revendications 1 à 8 où R et R' sont indépendamment hydrogène, méthyle, éthyle, propyle, isopropyle, n-butyle ou iso-butyle.

10. Composés selon les revendications 1 à 9 où A est phényle ou phényle substitué avec un ou deux substituants Q tels que définis dans la revendication 1.

11. Composés selon les revendications 1 à 10 où V est O, $S(O)_n$ ou $N—R^8$.

12. Composés selon la revendication 11 où V est O.

13. Les composés:

1'-phényl-spiro[cyclopentane-1,3'-(1,8)naphthyridine]-2',4'(1'*H*)-dione;

1'-phényl-spiro[cyclopentane-1,3'-quinoline]-2',4'-(1'*H*)-dione;

1'-(4-méthylphényl)spiro[cyclopentane-1,3'-(1,8)naphthyridine]-2',4'-(1'*H*)-dione;

1'-(4-chlorophényl)spiro[cyclopentane-1,3'-(1,8)naphthyridine]-2',4'-(1'*H*)-dione;

1'-(3,4-dichlorophényl)spiro[cyclopentane-1,3'-(1,8)naphthyridine]-2',4'-(1'*H*)-dione;

1'-(3-chlorophényl)spiro[cyclopentane-1,3'-(1,8)naphthyridine]-2',4'-(1'*H*)-dione;

1'-(3-méthoxyphényl)spiro[cyclopentane-1,3'-(1,8)naphthyridine]-2',4'-(1'*H*)-dione;

1'-(3-hydroxyphényl)spiro[cyclopentane-1,3'-(1,8)naphthyridine]-2',4'-(1'*H*)-dione;

1'-phénylspiro[cyclopentane-1,3'-quinoline]-2',4'-(1'*H*)-dione.

14. Les composés:

1-phényl-3',4',5',6'-tétrahydrospiro[1,8-naphthyridine-3,2'-(2*H*)pyran]-2,4-dione;

1-(3-méthoxyphényl)-3',4',5',6'-tétrahydro-spiro[1,8-naphthyridine-3,2'-(2*H*)pyran]-2,4-dione;

4,5-dihydro-1'-phényl-spiro[furan-2(3*H*),3'(2'*H*)(1,8)naphthyridine]-2',4'(1'*H*)-dione;

1-phényl-spiro[1,8-naphthyridine-3,2'-oxétane]-2,4-dione; and

1-(3-chlorophényl)-3',4',5',6'-tétrahydro-spiro[1,8-naphthyridine-3,2'-(2*H*)pyran]-2,4-dione.

15. Compositions pharmaceutiques comprenant comme ingrédient actif un composé tel que défini dans l'une quelconque des revendications 1 à 14 en mélange avec un support pharmaceutique acceptable.

16. Procédé pour la préparation de compositions pharmaceutiques telles que définies dans la revendication 15, qui comprend le mélange d'un composé tel que défini dans l'une quelconque des revendications 1 à 14 avec un support pharmaceutique acceptable.

17. Procédé pour la préparation de composés de formule générale I telle que définie dans la revendication 1, caractérisé en ce qu'un composé de formule générale IV

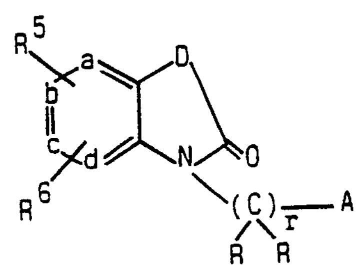

IV

où a, b, c, d, R, $R^5$, $R^6$, A et r sont tels que définis ci-dessus et D représente soit des groupements:

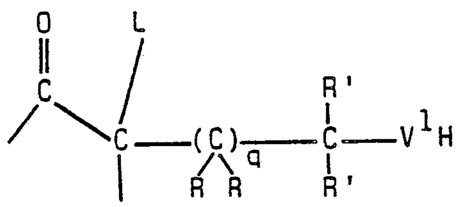

VI

ou

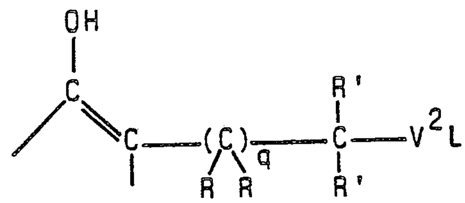

VII

où

L est un bon groupement de départ,

$V^1$ est O, $S(O)_n$ ou $N—R^8$,

$V^2$ est

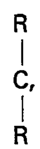

et

R, R' et q sont tels que définis ci-dessus,

est soumis à une condensation intramoléculaire produisant un composé de formule I où à la fois Y et Z sont oxygène, suivi, si on le souhaite, par le remplacement d'un ou des deux atomes d'oxygène à la fois, représentés par Y et Z, par du soufre.

**Revendications pour l'Etat contractant: AT**

1. Procédé pour la préparation de composés de formule générale I

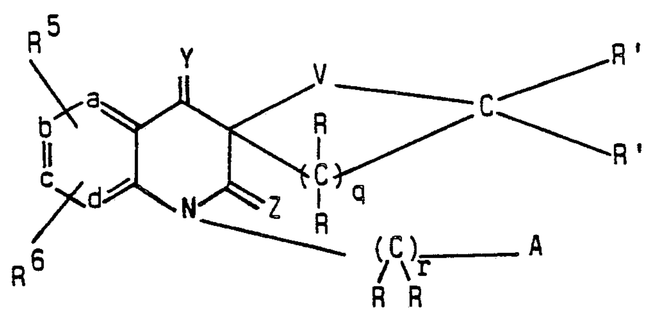

I

dans laquelle

deux des atomes a, b, c et d du noyau peuvent être CH ou N et les deux atomes restants représentent CH;

Y et Z représentent indépendamment O ou S;

V représente O, $S(O)_n$, $N—R^8$ ou

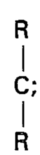

chaque R représente indépendamment un hydrogène, un alkyle de $C_1$ à $C_6$, $CH_2OH$, $COR_7$ (où $R^7$ représente un hydrogène ou un alkyle de $C_1$ à $C_6$) ou un hydroxy, à la condition que uniquement un groupement hydroxy soit attaché à un atome de carbone;

chaque R' indépendamment est défini comme pour R ci-dessus, excepté que lorsque V représente O, $S(O)_n$ ou $N—R^8$, R' ne peut pas être hydroxy;

$R^5$ et $R^6$ peuvent être les mêmes ou différents et sont hydrogène, alkyle ayant de 1 à 6 atomes de carbone, halogène, nitro, alkoxy ayant de 1 à 6 atomes de carbone, trifluorométhyle, alkylthio ayant de 1 à 6

atomes de carbone ou cyano; $R^8$ est hydrogène, alkyle ayant de 1 à 6 atomes de carbone, alcyle carboxylique ayant de 2 à 7 atomes de carbone, alkylsulfonyle ayant de 1 à 6 atomes de carbone, carboalkoxy ayant de 2 à 7 atomes de carbone, $CONH_2$, phényle ou pyridyle dont les deux derniers peuvent être substitués avec jusqu'à trois substituants, Q, où chaque Q est indépendamment hydroxy, alkyle ayant de 1 à 6 atomes de carbone, halogène, nitro, alcoxy ayant de 1 à 6 atomes de carbone, trifluorométhyle, cyano, cycloalkyle ayant de 3 à 7 atomes de carbone, alcényloxy ayant de 3 à 6 atomes de carbone, alkynyloxy ayant de 3 à 6 atomes de carbone, $S(O)_n$—$R^a$ [où n est défini ci-dessous et $R^a$ est un alkyle ayant de 1 à 6 atomes de carbone], $NHSO_2R^a$ [où $R^a$ est défini ci-dessous] $NHSO_2CF_3$, $SO_2NH_2$, $COR^b$ [où $R^b$ est OH, $NH_2$ ou $OR^a$ (où $R^a$ est défini ci-dessous)], O—B—$COR^b$ [où B est un alkylène ayant de 1 à 4 atomes de carbone et $R^b$ est défini ci-dessous], ou $NHCOR^c$ [où $R^c$ est un hydrogène, un alkyle ayant de 1 à 6 atomes de carbone, un alkoxy ayant de 1 à 6 atomes de carbone, $COR^d$ (où $R^d$ est hydroxy ou alcoxy ayant de 1 à 6 atomes de carbone) ou $NHR^e$ (où $R^e$ est hydrogène ou alkyle ayant de 1 à 6 atomes de carbones)];

n est 0, 1 ou 2;

r est 0, 1 ou 2;

q est un nombre entier de 1 à 5; et

A est phényle, naphtyle, indényle, indanyle, pyridyle, pyrimidyle, pyrazinyle, furyle, thiényle, imidazolyle, thiazolyle, ou oxazolyle, dont l'un quelconque peut être substitué avec jusqu'à trois substituants Q tels que définis ci-dessus, et les sels et les solvates de tels composés, caractérisé en ce qu'un composé de formule générale:

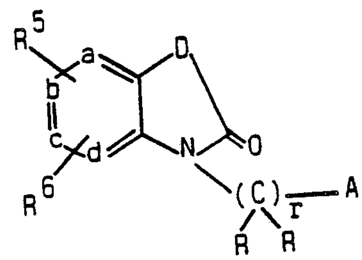

IV

où a, b, c, d, R, $R^5$, $R^6$, A et r sont tels que définis ci-dessus et D représente soit le groupe:

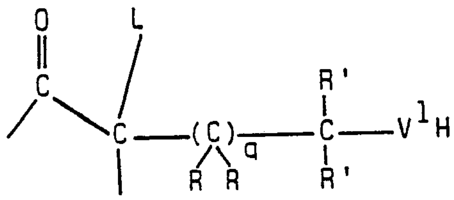

ou

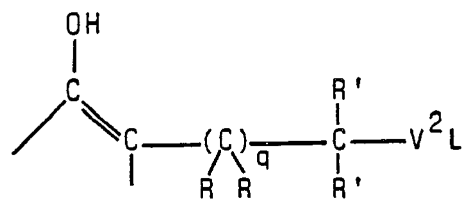

VI VII

où

L est un bon groupement de départ,

$V^1$ est O, $S(O)_n$ ou N—$R^8$,

$V^2$ est

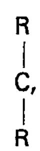

et

R, R' et q sont tels que définis ci-dessus,

est soumis à une condensation intramoléculaire produisant un composé de formule I où à la fois Y et Z sont oxygène, suivi, si on le souhaite, par le remplacement d'un ou des deux atomes d'oxygène à la fois, représentés par Y et Z, par du soufre.

2. Procédé selon la revendication 1, caractérisé en ce que la condensation intramoléculaire est accomplie en traitant le composé de formule I dans un solvant inerte avec une base.

3. Procédé selon la revendication 2, caractérisé en ce qu'une base organique est utilisée.

4. Procédé selon l'une quelconque des revendications 1 à 3, caractérisé en ce que la conversion des atomes d'oxygène représentés par X et/ou Y en soufre est accomplie par un traitement avec un sulfure approprié.

5. Procédé selon l'une quelconque des revendications 1 à 4, caractérisé en ce que le groupement de départ L est sélectionné parmi le brome, l'iode, le trifluoroacétoxy, le p-toluènesulfonyloxy et le méthanesulfonyloxy.

6. Procédé selon l'une quelconque des revendications 1 à 5, caractérisé en ce que les composés de formule I où Y et Z sont tous les deux oxygène, r est égal à zéro, a, b, c sont CH et d est N ou CH, q est 2, 3 ou 4 et A est phényle ou phényle substitué par un ou deux substituants Q [Q étant tel que défini dans la revendication 1] sont préparés.

7. Procédé selon l'une quelconque des revendications 1 à 6, caractérisé en ce que les composés:
1'-phényl-spiro[cyclopentane-1,3'-(1,8)naphthyridine]-2',4'(1'*H*)-dione;
1'-phényl-spiro[cyclopentane-1,3'-quinoline]-2',4'-(1'*H*)-dione;
1'-(4-méthylphényl)spiro[cyclopentane-1,3'-(1,8)naphthyridine]-2',4'-(1'*H*)-dione;
1'-(4-chlorophényl)spiro[cyclopentane-1,3'-(1,8)naphthyridine]-2',4'-(1'*H*)-dione;
1'-(3,4-dichlorophényl)spiro[cyclopentane-1,3'-(1,8)naphthyridine]-2',4'-(1'*H*)-dione;
1'-(3-chlorophényl)spiro[cyclopentane-1,3'-(1,8)naphthyridine]-2',4'-(1'*H*)-dione;
1'-(3-méthoxyphényl)spiro[cyclopentane-1,3'-(1,8)naphthyridine]-2',4'-(1'*H*)-dione;
1'-(3-hydroxyphényl)spiro[cyclopentane-1,3'-(1,8)naphthyridine]-2',4'-(1'*H*)-dione;
1'-phénylspiro[cyclopentane-1,3'-quinoline]-2',4'-(1'*H*)-dione;
1-phényl-3',4',5',6'-tétrahydrospiro[1,8-naphthyridine-3,2'-(2*H*)pyran]-2,4-dione;
1-(3-méthoxyphényl)-3',4',5',6'-tétrahydro-spiro[1,8-naphthyridine-3,2'-(2*H*)pyran]-2,4-dione;
4,5-dihydro-1'-phényl-spiro[furan-2(3*H*),3'(2'*H*)(1,8)naphthyridine]-2',4'(1'*H*)-dione;
1-phényl-spiro[1,8-naphthyridine-3,2'-oxétane]-2,4-dione; et
1-(3-chlorophényl)-3',4',5',6'-tétrahydro-spiro[1,8-naphthyridine-3,2'-(2*H*)pyran]-2,4-dione.

8. Procédé pour la préparation de compositions pharmaceutiques, caractérisé en ce qu'un composé tel que défini dans l'une quelconque des revendications 1 à 7 est mélangé avec un support pharmaceutique acceptable.